# EUROPEAN PATENT APPLICATION

(11) **EP 4 729 010 A1**
(43) Date of publication of application: **22.04.2026**
(21) Application number: 24823455.1
(22) Date of filing: 14.06.2024
(51) Int. Cl.: A61B 18/24, A61M 25/00, A61M 25/06, A61N 5/067

(54) **METALLIC GUIDE TUBE**

(30) Priority: 14.06.2023 JP 2023097559
(71) Applicant: Rakuten Medical, Inc., San Diego, California 92121 (US)
(72) Inventor: KANEBAKO, Hideki, Tokyo 158-0094 (JP)
(74) Representative: Meissner Bolte Partnerschaft mbB
(86) International application number: PCT/JP2024/021598
(87) International publication number: WO 2024/257842

(57) **Abstract**

The present invention is a metal guide tube that is deformable, has a shape-retention function for maintaining a deformed shape, and is configured to allow a medical instrument to be guided and inserted into the metal guide tube, and deform to allow the medical instrument to reach or approach a treatment target site.

## Description

### Technical Field

The present invention relates to a metal guide tube.

### Background Art

A treatment protocol exists that proceeds in two stages constituted by administration to a patient of a drug containing a complex formed of a photosensitive substance and a component that selectively accumulates in specific cells, and irradiation with light of a wavelength at which the photosensitive substance reacts. A photosensitive substance is a substance which reacts to light in a specific wavelength band. After administration of the drug and selective accumulation in specific cells, those cells are irradiated with light in a specific wavelength to activate the photosensitive substance and further cause necrosis of, or eliminate the specific cells through biochemical and physical processes. This treatment imposes a relatively small burden on patients owing to limited damage to cells other than specific cells and low adverse effects.

In this treatment, for example, one may insert a medical instrument for light irradiation through the mouth or nose and causes the instrument to reach or approach the target cells. Depending on locations of the specific cells, a route for inserting the medical instrument becomes complicated. In that case, the medical instrument cannot readily reach or approach the specific cells.

### Summary of Invention

### Technical Problem

The present invention provides a metal guide tube enabling a medical instrument to readily reach or approach a treatment target site.

### Solution to Problem

The present invention is a metal guide tube that is deformable, has a shape-retention function for maintaining a deformed shape, and is configured to allow a medical instrument to be guided and inserted into the metal guide tube, and deform to allow the medical instrument to reach or approach a treatment target site.

### Advantageous Effects of Invention

The present invention can provide a metal guide tube allowing a medical instrument to readily reach or approach a treatment target site.

### Brief Description of Drawings

FIG. 1 is an enlarged view showing an example of a cross section of a material constituting a metal tube according to embodiments.
FIG. 2 is an enlarged view showing an example of the cross section of the material constituting the metal tube according to embodiments.
FIG. 3 is an enlarged view showing an example of the cross section of the material constituting the metal tube according to embodiments.
FIG. 4 is an enlarged view showing an example of a surface of the material constituting the metal tube according to embodiments.
FIG. 5 is an enlarged picture showing an example of the metal tube according to embodiments.
FIG. 6 is a plan view of a metal guide tube according to embodiments.
FIG. 7 is a side view of the metal guide tube according to the embodiment of FIG. 6 (as seen from the right side of FIG. 6).

### Mode for Carrying Out the Invention

The following will describe embodiments of the present invention. The invention is not limited to the following descriptions. Various modifications or improvements may be made to the embodiments. Such modified or improved forms may also fall within the scope of the invention.

In the present specification, a distal end is an end introduced into the body lumen in the longitudinal axis direction of the metal guide tube, and a proximal end is the opposite end operated by hands.

### <First Embodiment>

The following will describe a metal guide tube according to the first embodiment.

The metal guide tube according to the first embodiment is deformable and has a shape-retention function for maintaining a deformed shape. The metal guide tube is configured to allow a medical instrument to be guided and inserted into the metal guide tube. Deforming the metal guide tube allows the medical instrument to be introduced to reach or approach treatment target sites.

The metal guide tube is deformable and has a shape-retention function for maintaining a deformed shape. The metal guide tube at least has the deformable distal end and the shape-retention function. The metal guide tube is deformable enough to be curved and bent in desired directions. Furthermore, the metal guide tube is preferably deformable enough to be curved and bent in any direction. This configuration enables the metal guide tube to be deformed into a shape that conforms to the position and the shape within the body lumen up to the treatment target site and facilitates the insertion. The metal guide tube is deformable by using tools such as forceps. The metal guide tube is more preferably deformable by hands without the use of tools. The metal guide tube preferably has a shape-retention function enough to maintain the deformed shape until the medical instrument reaches or approaches the treatment target site.

This metal guide tube can be deformed into a shape conforming to the position and shape within the body lumen up to the treatment target site and facilitating the insertion and can maintain this deformed shape. Thus, the metal guide tube allows the medical instrument inserted into the metal guide tube to be readily introduced to reach or approach treatment target sites.

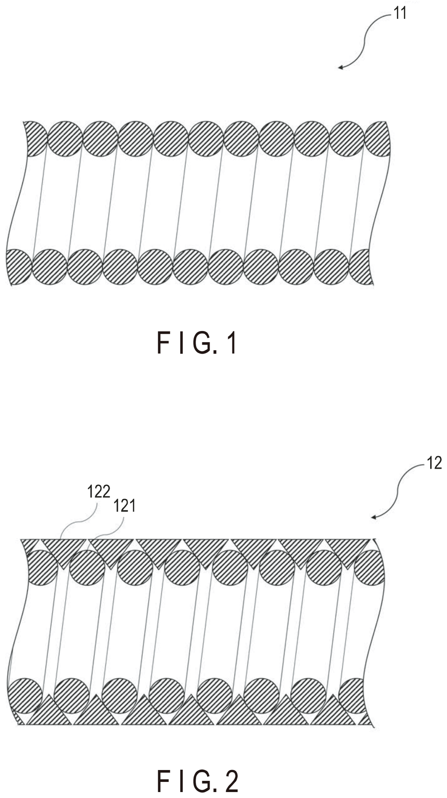

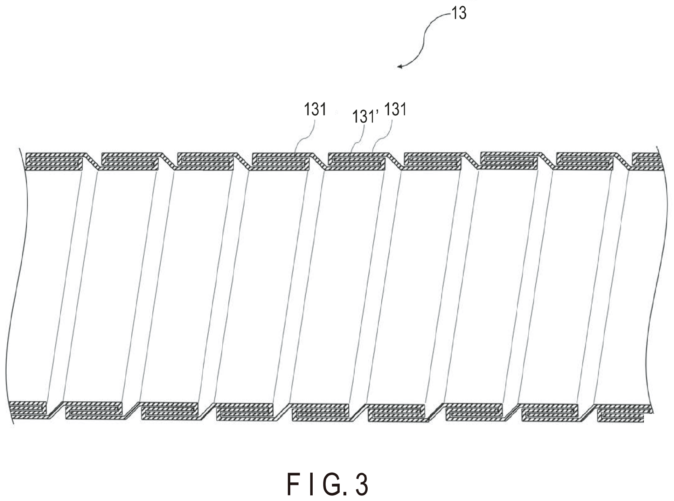

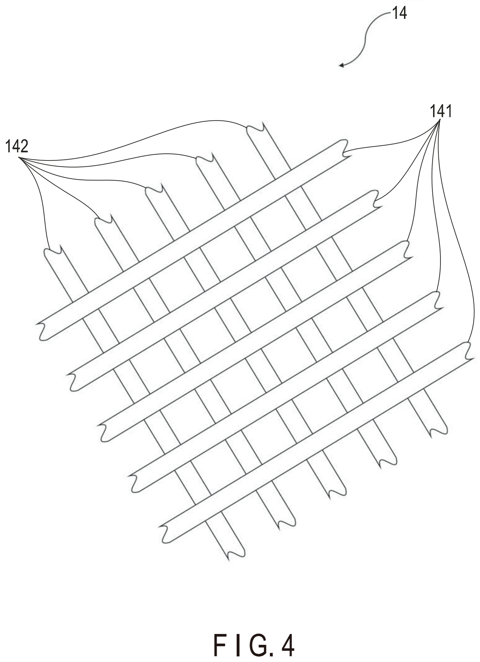

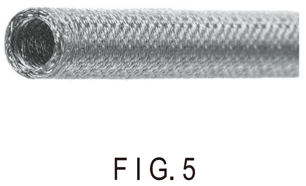

For example, this metal guide tube may be formed of a material formed by stacking metal wires in a mesh pattern. Alternatively, this metal guide tube may be formed of a material formed by arranging metal wires in a non-woven fabric form. Alternatively, this metal guide tube may be formed of a highly flexible metal (for example, aluminum, etc.). For example, the metal guide tube may be formed by spring-coiling metal wires and joining the wires together as shown in FIG. 1. Further, as shown in FIG. 2, the metal guide tube may be a so-called stand tube formed by interlocking wires 121 having a circular cross-sectional shape and wires 122 having a non-circular cross-sectional shape such as triangular. Furthermore, as shown in FIG. 3, the metal guide tube may be a so-called interlock tube formed by spring-coiling a flat metal wire that has an S-shaped cross-section and include bending portions 131 and 131' at both ends for the bending portions 131 and 131' of adjacent wires to interlock with each other. As described above, the metal guide tube is preferably a tube formed of a material formed by stacking metal wires in a mesh pattern. Such a tube has a good shape-retention function, resist collapse of the internal space when bent, and a simple structure, and requires relatively low manufacturing costs. For example, the following tube can be used as the above tube. As shown in FIG. 4, a stacked layer body 14 is formed. The stacked layer body 14 comprises a layer 141 formed by winding metal wires inclined in one direction around a columnar structure at a predetermined pitch, and a layer 142 formed by stacking and winding metal wires inclined in a direction different from the one direction around the columnar structure at a predetermined pitch on the layer 141. After joining the metal wires constituting the stacked layer body 14 by welding and the like, the columnar structure is removed to obtain the metal guide tube. FIG. 4 shows the stacked layer body 14 having two layers as an example. The number of layers is not limited to this example and can be changed according to the thickness of the metal guide tube. FIG. 5 shows a metal guide tube formed of a plurality of layers. Adjusting the number of the layers and the thickness of the metal guide tube can control bending rigidity of the metal guide tube. Furthermore, bonding one or more layers of metal foil to be interposed between the stacked bodies can adjust the bending rigidity, providing the metal guide tube with watertight and airtight functions. Furthermore, the bending rigidity of the metal guide tube can be freely varied by continuously or stepwise changing the number of layers along the longitudinal axis of the metal guide tube, providing parts for clamping and bonding metal foil having one layer or plurality of layers on at least part of the metal guide tube, or by combinations of these methods. Examples of such metal guide tubes include products like Fujifrape manufactured by Fuji Filter Co.

The metal guide tube is preferably formed of a metal material such as stainless steel. Examples of stainless steel include JIS standard SUS316L and SUS304.

The metal guide tube is configured to allow the medical instrument to be guided and inserted into the metal guide tube. For example, the metal guide tube is configured to allow the medical instrument to be inserted from the proximal end of the metal guide tube toward its distal end along the internal of the metal guide tube.

The inner diameter of the metal guide tube is greater than the diameter of the medical instrument. This configuration allows the medical instrument to be guided and inserted into the metal guide tube. Further, the inner diameter of the metal guide tube is preferably similar to the diameter of the medical instrument. This configuration allows the medical instrument to be securely held into the metal guide tube. When the medical instrument is BioBlade (registered trademark) Frontal Diffuser C or BioBlade (registered trademark) Frontal Diffuser H manufactured by Rakuten Medical, Inc. (described later), the inner diameter of the metal guide tube is preferably 2.5 mm or greater but close to 2.5 mm. Furthermore, the medical instrument may be a therapeutic optical fiber probe comprising an optical transmission structure capable of outputting light in a predetermined direction and a mirror structure positioned to receive light output from the optical transmission structure. This mirror structure reflects part of the received light required for treatment at a reflectance of 90% or more in a direction different from the predetermined direction and generates a light spot exhibiting a uniform light energy distribution across the entire light spot.

The metal guide tube is preferably configured such that the bending rigidity obtained by a three-point bending test (compliant with JISK 7171, span length 15 mm, test speed 1.0 mm/min, bending load when the indenter moves 1.5 mm) decreases stepwise or continuously from the proximal end to the distal end of the metal guide tube. Furthermore, the bending rigidity is preferably 35 N·mm² or greater within a 5 cm range from the distal end toward the proximal end of the metal guide tube and is preferably 50 N·mm² or greater within a range beyond that 5 cm range. For example, even when stress concentrates on a joint portion between a more deformable part and the other parts, the configuration in which the bending rigidity changes gradually or stepwise can prevent breaking of the metal guide tube.

The medical instrument can be any medical instruments that include a linear portion at least on a distal end side. Examples of the medical instrument include an optical fiber medical instrument comprising optical fibers used in a treatment protocol proceeding in the following two stages or a needle catheter into which the optical fiber is inserted. The two stages are constituted by administration to a patient of a drug containing a complex formed of a photosensitive substance and a component that selectively accumulates in specific cells, and irradiation with light of a wavelength at which the photosensitive substance reacts. The optical fiber medical instrument may be a medical instrument that includes an optical fiber for guiding laser light incident from a laser device, and an irradiation tip attached to the distal end of the optical fiber for irradiating the laser light from the distal end of the optical fiber at a specific angle and orientation. Examples of such optical fiber medical instruments include Rakuten Medical's BioBlade (registered trademark) Frontal Diffuser C, BioBlade (registered trademark) Frontal Diffuser H, and BioBlade (registered trademark) Cylindrical Diffuser. The needle catheter may be formed of laser-transmissive resin and having a distal end with a sharp and closed shape. Examples of this needle catheter include Rakuten Medical's BioBlade (registered trademark) needle catheter.

The therapeutic target site may be any cells that are in positions where the medical instruments are difficult to reach or approach. Examples of this therapeutic target site include specific cells within the body lumen located in such positions, such as cancer cells. For example, the body lumen includes cavities such as the oral cavity and the nasal cavity. Examples of such cancer cells include head and neck cancer cells. Examples of head and neck cancers include pharyngeal cancers such as nasopharyngeal cancer, oropharyngeal cancer, and hypopharyngeal cancer; laryngeal cancers such as glottic cancer, supraglottic cancer, and subglottic cancer; nasal cavity and paranasal sinus cancers such as maxillary sinus cancer; oral cancers such as tongue cancer; salivary gland cancer; and thyroid cancer.

### <Second Embodiment>

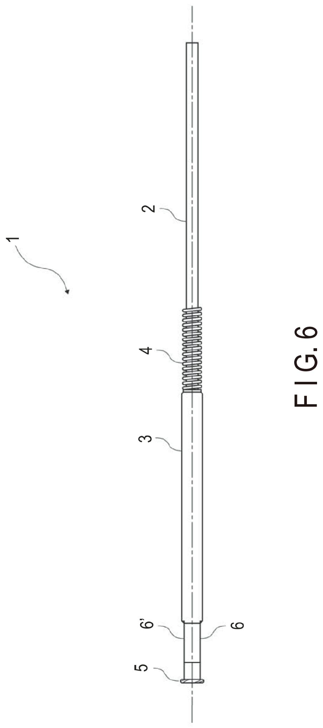

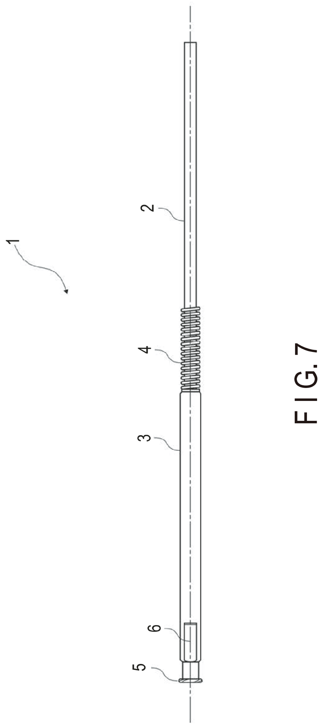

The following will describe a metal guide tube according to the second embodiment with reference to FIG. 6 and FIG. 7. Descriptions of configurations identical to those in the first embodiment are omitted by reference to the above description.

A metal guide tube 1 according to the second embodiment is deformable and has a shape-retention function for maintaining a deformed shape. The metal guide tube 1 is configured to allow a medical instrument to be guided and inserted into the metal guide tube 1. Deforming the metal guide tube 1 allows the medical instrument to be introduced to reach or approach treatment target sites. Further, the metal guide tube 1 according to the second embodiment is deformable and comprises a metal tube 2 having a shape-retention function for maintaining a deformed shape and a metal support tube 3 having high rigidity.

The metal guide tube 2 can be any metal tubes that are deformable and have a shape-retention function for maintaining a deformed shape. The metal tube may be the metal guide tube according to the first embodiment.

The metal support tube 3 allows an operator to stably grasp the metal guide tube 1. The distal end of the metal support tube 3 is connected to the proximal end of the metal tube 2. The metal support tube 3 may be connected to the proximal end of the metal tube 2, for example, by brazing, arc welding, laser welding, and the like.

The metal support tube 3 need not be deformable. Alternatively, the metal support tube 3 may be deformable and have a shape-retention function. In the latter case, the metal support tube 3 preferably has higher rigidity than the metal tube 2. The metal support tube 3 having higher rigidity than the metal tube 2 allows an operator to stably grasp the metal guide tube 1.

The metal support tube 3 is preferably formed of a metal material such as a stainless steel. Examples of the stainless steel include JIS standard 316L and SUS304.

The metal support tube 3 may include a structure for being gripped by a fixture or the like. The shape and number of such structures are not particularly limited as long as the metal support tube 3 can be gripped by a fixture or the like. As one example of such a structure, FIG. 6 shows two structures 6 and 6' provided on the respective side surfaces of the metal support tube 3 and have a plane parallel to the long axis of the metal guide tube 1.

The metal guide tube 1 may further include a spring 4 that protects a joint portion between the metal tube 2 and the metal support tube 3. This configuration can mitigate strain on the joint portion when the metal guide tube 1 is bent abruptly, preventing breakage of the joint portion.

The spring 4 is preferably formed of a metal material such as stainless steel. Examples of the stainless steel include JIS standard 316L and SUS304.

The metal guide tube 1 may also include a connector 5 provided at the proximal end of the metal support tube 3. The connector 5 may be formed by a known luer taper or the like capable of connecting adapters such as commercially available Tuohy-Borst valve adapters (TBA) and syringes. The connector 5 can connect adapters and syringes to the metal guide tube 1.

The metal guide tubes according to the first and second embodiments described above are deformable and possess a shape-retention function to maintain their deformed shape. Further, the metal guide tube is configured to allow the medical instrument to be guided and inserted into the metal guide tube. Deforming the metal guide tube allows the medical instrument to be introduced to reach or approach treatment target sites. Such a metal guide tube allows a medical instrument to readily reach or approach a treatment target site, even when the target site is located in a position that the medical instrument cannot readily reach or approach.

The following will describe a method for introducing a medical instrument into a living body using the metal guide tube according to the first and second embodiments.

The method for introducing a medical instrument into a living body using the metal guide tube according to the first and second embodiments comprises: (a) inserting and guiding the medical instrument into the metal guide tube, which is deformable and has a shape-retention function; and (b) introducing the metal guide tube into the living body and deforming the metal guide tube for the medical instrument to reach or approach the treatment target site.

In the above step (a), the medical instrument is guided and inserted into the metal guide tube. When the metal guide tube 1 shown in FIG. 6 is used as the metal guide tube, the step (a) may be performed as follows. The distal end of the medical instrument is inserted into the metal guide tube 1 via the proximal end of the metal support tube 3. The medical instrument is inserted toward the distal end of the metal guide tube 1 while the distal end of the medical instrument being guided along the interior of the metal guide tube 1. The insertion continues until at least the distal end of the medical instrument protrudes from the distal end of the metal guide tube 1.

In the above step (b), the metal guide tube is inserted within the body lumen to deform to allow the medical instrument to reach or approach the treatment target site. The body lumen includes, for example, cavities such as the oral cavity and the nasal cavity. For example, the metal guide tube can be inserted from mouth and nose to the above body lumen.

The metal guide tube is deformed into a shape conforming to the shape of the body lumen leading to the treatment target site and enabling easy insertion. The metal guide tube may be deformed by hands or a jig and the like.

The insertion and deformation of the metal guide tube may be performed in either order. The insertion and deformation may be repeated until the medical instrument reaches or approaches the treatment target site.

For example, when the treatment target site is located in the oral cavity, the above step (b) of inserting and deforming the metal guide tube within the body lumen can be performed as follows. First, the metal guide tube is deformed into a shape conforming to the shape of the oral cavity leading from the patient's mouth to the treatment target site and enabling easy insertion. Then, the metal guide tube is introduced from the patient's mouth toward the treatment target site to cause the distal end of the metal guide tube to approach the treatment target site. These processes allow at least the distal end of the medical instrument exposed from the distal end of the metal guide tube to reach or approach the treatment target site.

As described above, even when the treatment target site is located in a position that the metal guide tube cannot readily reach or approach, the metal guide tubes according to the first embodiment and the second embodiment allow the medical instrument to readily reach or approach the treatment target site.

Th following will describe the present invention in more detail by describing a more specific embodiment as an example. Note that the present invention is not limited to the following examples.

A guide tube equivalent to the metal guide tube 1 shown in FIG. 6 is prepared. An optical fiber medical instrument used in a treatment protocol proceeding in the following two stages or a needle catheter into which the optical fiber is inserted are prepared. The two stages are constituted by administration to a patient of a drug containing a complex formed of a photosensitive substance and a component that selectively accumulates in specific cells, and irradiation with light of a wavelength at which the photosensitive substance reacts. The optical fiber medical instrument or the needle catheter is guided and inserted into the metal guide tube. The metal guide tube is inserted within the body lumen to deform to allow the medical instrument or the needle catheter to reach or approach the treatment target site. As a result, the optical fiber medical instrument or the needle catheter readily reaches or approaches the treatment target site located at the position that the optical fiber medical instrument or the needle catheter does not readily reach or approach.

### Reference Signs List

1 ... Metal guide tube, 2 ... Metal tube, 3 ... Metal support tube, 4 ... Spring, 5... Connector.

## Claims

1. A metal guide tube that is deformable, has a shape-retention function for maintaining a deformed shape and is configured to:
allow a medical instrument to be guided and inserted into the metal guide tube; and
deform to allow the medical instrument to reach or approach a treatment target site.

2. The metal guide tube of claim 1, further comprising:
a metal tube deformable and having a shape-retention function for maintaining a deformed shape; and
a metal support tube having high rigidity.

3. The metal guide tube of claim 1, wherein
the metal guide tube has a structure in which metal wires are stacked in a mesh pattern or a structure in which the metal wires are arranged in a non-woven fabric form.

4. The metal guide tube of claim 1 wherein
the medical instrument is an optical fiber medical instrument used in a treatment protocol proceeding in two stages or a needle catheter into which the optical fiber is inserted, the two stages being constituted by administration to a patient of a drug containing a complex formed of a photosensitive substance and a component that selectively accumulates in specific cells, and irradiation with light of a wavelength at which the photosensitive substance reacts.

5. The metal guide tube of claim 1, wherein
bending rigidity of a metal tube of the metal guide tube decreases stepwise or continuously from a proximal end to a distal end of the metal guide tube.

6. The metal guide tube of claim 2, wherein
the metal support tube is connected to the metal tube.

7. The metal guide tube of claim 6, further comprising:
a member for protecting a joint portion between the metal guide tube and the metal support tube.
